# EUROPÄISCHE PATENTSCHRIFT

(11) **EP 2 291 163 B1**
(45) Veröffentlichungstag und Bekanntmachung des Hinweises auf die Patenterteilung: **05.12.2012**
(21) Anmeldenummer: 09765619.3
(22) Anmeldetag: 18.06.2009
(51) Int. Cl.: A61J 1/00, A61K 9/19, F26B 5/06

(54) **BEHÄLTER FÜR MEDIZINISCHE PRODUKTE UND VERFAHREN ZU DESSEN HERSTELLUNG**
CONTAINER FOR MEDICAL PRODUCTS AND METHOD FOR PRODUCTION OF SAID CONTAINER
CONTENANT POUR PRODUITS MÉDICAMENTEUX ET SON PROCÉDÉ DE FABRICATION

(30) Priorität: 19.06.2008 DE 102008030273
(43) Veröffentlichungstag der Anmeldung: 09.03.2011
(73) Patentinhaber: Arzneimittel GmbH Apotheker Vetter & Co. Ravensburg, 88212 Ravensburg (DE)
(72) Erfinder: BÖTTGER, Frank, 88214 Ravensburg (DE); BÖBST, Benjamin, 88441 Mittelbiberach (DE); KOCH, Patricia, 89073 Ulm (DE)
(74) Vertreter: Gleiss, Alf-Olav
(86) Internationale Anmeldenummer: PCT/EP2009/004385
(87) Internationale Veröffentlichungsnummer: WO 2009/153039

(56) Entgegenhaltungen:
- US-A- 3 240 328
- US-A- 3 269 905
- US-A- 3 337 041

## Beschreibung

Die Erfindung betrifft einen Behälter für medizinische Produkte gemäß dem Oberbegriff von Anspruch 1.

Solche Behälter sind bekannt. Sie weisen typischerweise eine Kammer auf, in der sich ein Wirk- und/oder Hilfsstoff befindet. Dieser Wirk- und/oder Hilfsstoff kann als Festphase -beispielweise gefriergetrocknet- vorliegen, oder auch in Form einer Lösung. Sollen mehrere Wirk- und/oder Hilfsstoffe einem Patienten gemeinsam verabreicht werden, ist es vorteilhaft, diese erst kurz vor der Verabreichung miteinander zu vermischen. Auf diese Weise kann häufig eine längere Haltbarkeit der Stoffe erzielt werden, insbesondere, wenn die Stoffe miteinander reagieren können.

In bekannten Behältern seten Zum Beispiel die Dokumente US-A-3 337 041, US-A-3 240 328 oder US-A-3269905, sind hierfür verschiedene Kammern vorgesehen, wobei in jeder der voneinander getrennten Kammem jeweils ein Wirk- und/oder Hilfsstoff separat vorliegt. Bekannt sind beispielsweise sogenannte Doppelkammersysteme, bei denen zwei Kammem voneinander getrennt vorliegen. Dabei sind Mittel vorgesehen, die es ermöglichen, die beiden Kammern kurz vor der Verabreichung des Medikaments miteinander zu verbinden, sodass eine durch Durchmischung der zuvor getrennt vorliegenden Komponenten stattfinden kann. In einem zweiten Schritt wird eine Dosis des durchmischten Medikaments dem Patienten verabreicht.

Nachteilig an den bekannten Systemen ist, dass bei komplexen Zusammensetzungen eines Medikaments für jeden Wirk- und/oder Hilfsstoff eine separate Kammer vorgesehen sein muss. Dies bedeutet, dass entweder mehrere einzelne Behälter mit den verschiedenen Wirk- und/oder Hilfsstoffen bereitgehalten werden müssen, oder dass ein sehr komplexes Behältnis bereitgestellt werden muss, das über mehrere miteinander verbindbare Kammern verfügt.

Aufgabe der Erfindung ist es daher, einen Behälter bereitzustellen, der die genannten Nachteile nicht aufweist.

Diese Aufgabe wird gelöst durch einen Behälter mit den Merkmalen des Anspruchs 1. Der Behälter für medizinische Produkte weist mindestens eine Kammer auf und zeichnet sich dadurch aus, dass mindestens zwei gefriergetrocknete Wirk- und/oder Hilfsstoffe gemeinsam in der mindestens einen Kammer vorliegen. Dadurch, dass die Wirk- und/oder Hilfsstoffe in gefriergetrockneter Form vorliegen, sind die molekularen Komponenten immobilisiert und entsprechend reaktionsträge. Das bedeutet, dass die Wirk- und/oder Hilfsstoffe selbst dann nur sehr langsam miteinander reagieren, wenn die gefriergetrockneten Substanzen innig miteinander vermischt vorliegen. Selbst in einer so ungünstigen Aufbewahrungsform -nämlich der innigen Durchmischung- sind die Stoffe also deutlich länger haltbar, als wenn sie gemeinsam in Lösung vorlägen.

Besonders bevorzugt wird ein Behälter, bei dem vorgesehen ist, dass die mindestens zwei Wirk- und/oder Hilfsstoffe in Schichten geordnet vorliegen. In diesem Fall berühren sich die einzelnen Komponenten lediglich an den Flächen, mit denen sie aneinander anliegen. Insofern ist eine Reaktion zwischen den Komponenten nur noch in sehr eingeschränktem Maße möglich. Die Haltbarkeit wird daher gegenüber einer innigen Durchmischung nochmals deutlich verbessert.

Bevorzugt wird auch ein Behälter, bei dem die mindestens zwei Wirk- und/oder Hilfsstoffe durch mindestens einen gefriergetrockneten Neutralstoff voneinander getrennt vorliegen. Hier wird angesprochen, dass ein gefriergetrockneter Neutralstoff die einzelnen Wirk- und/oder Hilfsstoffe räumlich voneinander trennt. Die aktiven Komponenten, also die Wirk- und/oder Hilfsstoffe, berühren sich nicht untereinander, sondern haben lediglich Kontakt mit dem Neutralstoff. Der Neutralstoff ist dabei so gewählt, dass er mit den aktiven Komponenten nur sehr langsam, vorzugsweise überhaupt nicht reagiert. Auf diese Weise kann die Haltbarkeit nochmals deutlich verbessert werden.

Besonders bevorzugt wird in diesem Zusammenhang ein Behälter, der sich dadurch auszeichnet, dass die mindestens zwei Wirk- und/oder Hilfsstoffe in Schichten geordnet vorliegen, und durch mindestens eine Schicht eines gefriergetrockneten Neutralstoffs voneinander getrennt sind. In diesem Fall liegt also eine definierte Schichtung vor, wobei zwischen je zwei Schichten aktiver Komponenten jeweils mindestens eine Schicht eines gefriergetrockneten Neutralstoffs angeordnet ist, sodass die aktiven Komponenten sich untereinander nicht berühren. Auf diese Weise ist eine sehr lange Haltbarkeit der aktiven Komponenten gegeben.

Der Behälter kann prinzipiell ein beliebiger Behälter für medizinische Produkte sein. Bevorzugt wird aber, dass der Behälter eine Spritze, Karpule, ein Doppel- oder Mehrkammersystem, eine Phiole, ein Infusionsbeutel oder eine Infusionsflasche ist. Wenn es sich bei dem Behälter um ein Einkammersystem handelt, muss vor der Verabreichung ein Lösungsmittel in den Behälter eingebracht werden, um die aktiven Komponenten aufzulösen. Die Lösung kann dann beispielsweise auf eine Spritze aufgezogen und dem Patienten verabreicht werden. Selbstverständlich ist es auch möglich, dass der Patient die auf diese Weise angefertigte Lösung trinkt oder äußerlich anwendet, beispielsweise auf die Haut aufträgt. In Mehrkammersystemen liegt dagegen üblicherweise das Lösungsmittel bereits in einer separaten Kammer vor. In diesem Fall müssen nur die Kammer mit dem Lösungsmittel und die Kammer mit den aktiven Komponenten in Fluidverbindung gebracht werden, sodass das Lösungsmittel die aktiven Komponenten auflösen kann. Anschließend kann die Lösung dem Patienten verabreicht werden.

Weitere Ausgestaltungen des Behälters ergeben sich aus den Unteransprüchen.

Aufgabe der Erfindung ist es weiterhin, ein Verfahren zur Herstellung eines genannten Behälters anzugeben.

Zur Lösung dieser Aufgabe wird ein Verfahren vorgeschlagen, mit dem ein erfindungsgemäßer Behälter herstellbar ist und das die in Anspruch 6 genannten Merkmale aufweist. Es umfasst die folgenden Schritte, die in ihrer Reihenfolge auch variiert werden können.

Es wird ein Behälter für medizinische Produkte bereitgestellt, der mindestens eine Kammer aufweist. In diese wird die Lösung eines ersten Wirk- und/oder Hilfsstoffs eingefüllt und schockgefroren. Danach wird eine weitere Lösung eines Wirk- und/oder Hilfsstoffs in die mindestens eine Kammer eingefüllt, wonach auch diese weitere Lösung schockgefroren wird. Die beiden letzten Schritte -also das Einfüllen einer weiteren Lösung sowie das Schockgefrieren der weiteren Lösung- können dabei beliebig oft wiederholt werden, bis eine gewünschte Anzahl von Wirk- und/oder Hilfsstoffen in der mindestens einen Kammer des Behälters vorliegt. Auf diese Weise entstehen Schichten schockgefrorener Substanzen, die typischerweise übereinander angeordnet sind. Nach Beendigung des Einfüllvorgangs werden die gefrorenen Lösungen gemeinsam in dem Behälter gefriergetrocknet. Dabei sublimiert der Lösungsmitteldampf aus den weiter unten gelegenen Schichten durch die weiter oben gelegenen Schichten hindurch.

Besonders bevorzugt wird auch ein Verfahren, das sich dadurch auszeichnet, dass nach dem Schockgefrieren einer Lösung eines Wirk- und/oder Hilfsstoffes zunächst ein Neutralstoff, vorzugsweise eine Lösung eines Neutralstoffs, in die mindestens eine Kammer eingefüllt wird. Dieser Neutralstoff oder dessen Lösung wird sodann schockgefroren, bevor die nächste Lösung eines Wirk- und/oder Hilfsstoffes in die mindestens eine Kammer eingefüllt wird. Auf diese Weise entstehen Schichten von Neutralstoffen, die einzelne Schichten der aktiven Komponenten voneinander trennen können.

Die Erfindung wird im Folgenden anhand der Zeichnung näher erläutert. Es zeigen:
- Figur 1: eine schematische Ansicht eines als Phiole ausgebildeten Behälters und
- Figur 2: einen als Doppelkammersystem ausgebildeten Behälter.

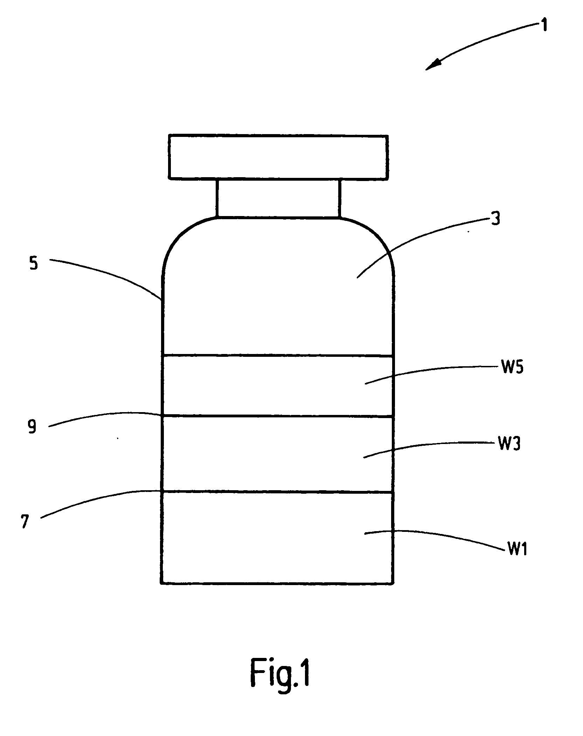

Figur 1 zeigt einen Behälter 1 für medizinische Produkte. Der Behälter 1 weist eine Kammer 3 auf, die zur Aufnahme von medizinischen Produkten geeignet ist. Die Kammer 3 wird von der Außenwand 5 des Behälters umschlossen, sodass der dargestellte Behälter 1 nur eine einzige Kammer 3 aufweist. Es ist auch möglich, in dem Behälter 1 mehrere Kammern 3 voneinander abzutrennen, indem beispielsweise Zwischenböden in die Kammer 3 eingebracht werden.

In der Kammer 3 liegen drei verschiedene Wirk- und/oder Hilfsstoffe W1, W3 und W5 gefriergetrocknet und in Schichten übereinander angeordnet vor. Es ist auch möglich, die Wirk- und/oder Hilfsstoffe -im Folgenden auch aktive Komponenten genannt- W1, W3 und W5 in anderer geometrischer Anordnung anzuordnen. Insbesondere können die einzelnen gefriergetrockneten aktiven Komponenten auch zermahlen oder zerstoßen und miteinander vermischt werden. Hierbei wird jedoch die Fläche, an der sich die aktiven Komponenten W1, W3 und W5 untereinander berühren, vergrößert, sodass möglicherweise stattfindende Reaktionen beschleunigt ablaufen. Im Sinne der Haltbarkeit der aktiven Komponenten W1, W3 und W5 ist es daher günstig, diese geschichtet über oder nebeneinander anzuordnen. In diesem Fall berühren sich die aktiven Komponenten W1, W3 und W5 nur an Berührungsflächen 7 und 9, wodurch Reaktionen zwischen den Komponenten W1 und W3 einerseits und den Komponenten W3 und W5 andererseits nur mit deutlich herabgesetzter Reaktionsgeschwindigkeit ablaufen können. Eine Reaktion der Komponente W1 mit der Komponente W5 ist ausgeschlossen, da diese durch die Komponente W3 räumlich voneinander getrennt sind.

Der Behälter 1 ist hier als Phiole dargestellt, er kann aber auch eine Spritze, Karpule, ein Doppel- oder Mehrkammersystem, ein Infusionsbeutel oder eine Infusionsflasche sein. Wesentlich ist nur, dass mindestens zwei gefriergetrocknete Wirk- und/oder Hilfsstoffe W1, W3, W5 in mindestens einer Kammer 3 gemeinsam vorliegen.

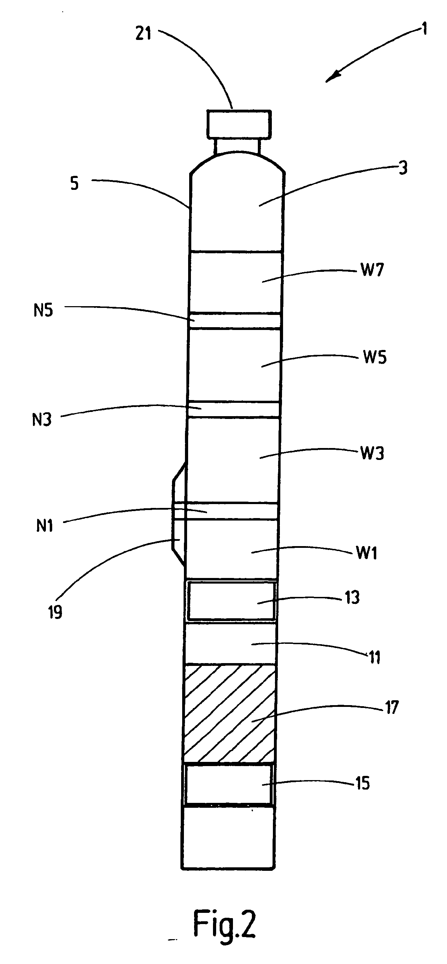

Figur 2 zeigt ein weiteres Ausführungsbeispiel eines Behälters 1 für medizinische Produkte, das als Doppelkammersystem ausgebildet ist. Gleiche und funktionsgleiche Elemente sind mit gleichen Bezugszeichen versehen, sodass insofern auf die vorangegangene Beschreibung verwiesen wird.

Der Behälter 1 weist hier neben der ersten Kammer 3 eine zweite Kammer 11 auf. Beide Kammern werden durch die Außenwand 5 des Behälters 1 begrenzt. Die erste Kammer 3 ist gegenüber der zweiten Kammer 11 durch einen Mittelstopfen 13 abgeteilt, der in dem Behälter 1 verlagerbar angeordnet ist. Die zweite Kammer 11 wird zum -vom Betrachter von Figur 2 aus gesehen- unteren Ende des Behälters 1 hin durch einen Endstopfen 15 abgeschlossen, der ebenfalls verlagerbar in dem Behälter 1 angeordnet ist. Die Stopfen 13 und 15 schließen dabei dicht mit der Außenwand 5 des Behälters 1 ab, können aber auf dieser gleiten, sodass sie verlagerbar sind.

In der ersten Kammer 3 sind im vorliegenden Ausführungsbeispiel vier verschiedene Wirk- und/oder Hilfsstoffe W1, W3, W5 und W7 in gefriergetrockneter Form übereinander geschichtet. Im Gegensatz zu dem in Figur 1 gezeigten Ausführungsbeispiel berühren sich die aktiven Komponenten W1, W3, W5 und W7 hier allerdings nicht direkt, sondern es sind Schichten gefriergetrockneter Neutralstoffe oder gefriergetrockneter Lösungen von Neutralstoffen N1, N3 und N5 zwischen den aktiven Komponenten W1, W3, W5 und W7 angeordnet. Daher gibt es keine Flächen, an denen sich je zwei aktive Komponenten W1, W3, W5 oder W7 berühren, sondern jede Fläche einer aktiven Komponente W1, W3, W5 oder W7 berührt entweder eine Wandung der Kammer 3 oder eine Außenfläche einer gefriergetrockneten Neutralkomponente N1, N3 oder N5. Auf diese Weise wird ein Kontakt zwischen den aktiven Komponenten W1, W3, W5 und W7 vollständig vermieden, sodass chemische oder biochemische Reaktionen zwischen den aktiven Komponenten W1, W3, W5 oder W7 ausgeschlossen sind. Dies bedingt eine sehr lange Haltbarkeit der aktiven Komponenten W1, W3, W5 und W7. Prinzipiell können die aktiven Komponenten W1, W3, W5 und W7 sowie die Neutralkomponenten N1, N3 und N5 auch in einer anderen als der dargestellten geometrischen Anordnung vorliegen. Wichtig ist nur, dass ein direkter Kontakt zwischen Flächen der aktiven Komponenten W1, W3, W5 und W7 dadurch vermieden wird, dass jeweils eine Neutralkomponente N1, N3 und N5 zwischen solchen Flächen der aktiven Komponenten W1, W3, W5 und W7 angeordnet ist, die sich ansonsten berühren würden.

Die zweite Kammer 11 umfasst ein Lösungsmittel 17, das in der Lage ist, zumindest die aktiven Komponenten W1, W3, W5 und W7 zu lösen. Vorzugsweise können auch die Neutralkomponenten N1, N3 und N5 von dem Lösungsmittel 17 gelöst werden. Im dargestellten Lagerzustand des Behälters 1 sind die erste Kammer 3 und die zweite Kammer 11 durch einen Mittelstopfen 13 voneinander getrennt, sodass das Lösungsmittel 17 nicht in Kontakt mit den Komponenten W1, W3, W5, W7, N1, N3 und N5 kommen kann. Kurz vor der Verabreichung des Medikaments an einen Patienten kann das Lösungsmittel 17 jedoch von der Kammer 11 in die Kammer 3 eingeleitet werden, um die dort enthaltenen Komponenten W1, W3, W5, W7, N1, N3 und N5 zu lösen.

Hierzu ist ein Bereich größeren Durchmessers an der Außenwand 5 des Behälters 1 vorgesehen, der eine Erstreckung entlang einer Längsachse des Behälters 1 aufweist, die größer ist als die Erstreckung des Mittelstopfens 13 entlang derselben Achse. Hierdurch kann der Bereich größeren Durchmessers als Bypass 19 wirken. Dabei überdeckt der Bereich größeren Durchmessers in Umfangsrichtung des Behälters 1 nur einen kleinen Winkelbereich, sodass der Mittelstopfen 13 auch im Bereich des Bypasses 19 durch die Außenwand 5 des Behälters 1 sicher geführt wird.

Soll das Medikament einem Patienten verabreicht werden, wird folgendermaßen vorgegangen: Zunächst wird der Endstopfen 15 in Richtung des -vom Betrachter von Figur 2 aus gesehen- oberen Endes des Behälters 1 verlagert, sodass über die hierdurch in die zweite Kammer 11 eingeleiteten Druckkräfte auch der Mittelstopfen 13 in die gleiche Richtung verlagert wird. Der Mittelstopfen 13 wird dabei solange verlagert, bis er sich in einem mittleren Bereich des Bypasses 19 befindet, sodass die erste Kammer 3 mit der zweiten Kammer 11 über den Bypass 19 verbunden ist. Wird nun der Endstopfen 15 weiter in dieselbe Richtung verlagert, umfließt das Lösungsmittel 17 den Mittelstopfen 13 und gelangt auf diese Weise in die erste Kammer 3. Dort löst das Lösungsmittel 17 zumindest die aktiven Komponenten W1, W3, W5 und W7 und vorzugsweise auch die Neutralkomponenten N1, N3 und N5. Bei weiterer Verlagerung des Endstopfens 15 in Richtung des Mittelstopfens 13 gibt es eine Position, in der sich die beiden Stopfen 13, 15 berühren. Wird nun der Endstopfen 15 weiter in den -vom Betrachter aus gesehen- oberen Bereich des Behälters 1 verlagert, nimmt er den Mittelstopfen 13 mit, sodass auch dieser in dieselbe Richtung verlagert wird. Hierbei wird die nun in der ersten Kammer 3 vorliegende Lösung in Richtung des Kopfendes 21 des Behälters 1 gedrückt. Dort kann eine Öffnung vorgesehen sein, durch die die Lösung entnehmbar ist. Beispielsweise kann das Kopfende 21 des Behälters 1 mit einer Kanüle versehen werden, durch welche die Lösung einem Patienten injiziert werden kann. Es ist aber auch denkbar, dass der Patient die Lösung der ersten Kammer 3 entnimmt und trinkt oder äußerlich anwendet, oder dass die Lösung dem Patienten als Einlauf verabreicht wird. Auch andere Arten der in der Medizin üblichen Verabreichung eines Medikaments sind möglich. Wesentlich ist, dass zunächst mehrere Wirk- und/oder Hilfsstoffe gefriergetrocknet gemeinsam in einer Kammer vorliegen, sodass einerseits komplizierte Mehrkammersysteme mit separaten Kammern für jede aktive Komponente vermieden werden, und andererseits die aktiven. Komponenten keine chemische oder biochemische Reaktion miteinander eingehen können. Erst kurz vor Verabreichung des Medikaments an einen Patienten sollen die aktiven Komponenten in einem Lösungsmittel gelöst und damit in einen verabreichbaren Zustand gebracht werden.

Im Folgenden soll das Verfahren zur Herstellung eines erfindungsgemäßen Behälters näher erläutert werden. Zunächst wird ein Behälter 1 bereitgestellt, der mindestens eine Kammer 3 aufweist. Es wird dann eine Lösung eines ersten Wirk- und/oder Hilfsstoffs W1 in die Kammer 3 eingefüllt. Die Lösung wird dann schockgefroren. Dies kann beispielsweise auf einer Tiefkühlstraße, in einem Bad mit flüssigem Stickstoff oder in ähnlichen Vorrichtungen erfolgen. Wesentlich ist nur, dass die erste Lösung eingefroren wird. Während des folgenden Prozesses muss vermieden werden, dass die gefrorene Lösung wieder auftaut. Der Behälter 1 muss daher während des gesamten weiteren Verfahrens auf einer Temperatur gehalten werden, die niedriger ist als der Schmelzpunkt der ersten Lösung.

Nach dem Gefrieren der ersten Lösung wird eine zweite Lösung eines Wirk- und/oder Hilfsstoffs W3 in die Kammer 3 eingebracht. Diese Lösung wird dann möglichst rasch schockgefroren, sodass an der Grenzfläche zwischen der ersten und der zweiten Lösung kein nennenswerter Schmelzprozess stattfinden kann. Auch die zweite gefrorene Lösung darf während des weiteren Verfahrens nicht schmelzen, sodass der Behälter 1 bei einer Temperatur gehalten werden muss, die niedriger liegt als der Schmelzpunkt der zweiten Lösung. Generell wird der Behälter 1 vorzugsweise während des gesamten Verfahrens auf einer Temperatur gehalten, die tiefer ist, als der niedrigste Schmelzpunkt der gefrorenen Lösungen, die in die Kammer 3 des Behälters 1 eingebracht werden sollen.

In dem Ausführungsbeispiel gemäß Figur 1 wird auf die gefrorene zweite Lösung noch eine dritte Lösung eines Wirk- und/oder Hilfsstoffs W5 aufgebracht, die ebenfalls schockgefroren wird. Es liegen nun also drei schockgefrorene Lösungen übereinander vor. Selbstverständlich ist es möglich nur zwei schockgefrorene Lösungen übereinander anzuordnen. Es ist aber auch möglich mehr als drei schockgefrorene Lösungen übereinander anzuordnen. Wie viele schockgefrorene Lösungen übereinander in der Kammer 3 des Behälters 1 angeordnet werden, ist ausschließlich durch die gewünschte Wirkung beim Patienten einerseits und die chemische beziehungsweise biochemische Verträglichkeit der Wirk- und/oder Hilfsstoffe untereinander bestimmt. So ist es beispielsweise auch möglich, mehrere nicht miteinander reagierende Wirk- und/oder Hilfsstoffe W1, W3, W5 in derselben Lösung vorzulegen und schockzugefrieren, während weitere, reaktive Wirk- und/oder Hilfsstoffe W1, W3, W5 in separaten schockgefrorenen Lösungen angeordnet werden. Wesentlich ist, dass eine Trennung von Wirk- und/oder Hilfsstoffen W1, W3, W5 stattfindet, die bei Kontakt miteinander chemisch oder biochemisch reagieren würden.

Wenn alle gewünschten Lösungen schockgefroren in dem Behälter 1 vorliegen, wird dieser in eine Vorrichtung zum Gefriertrocknen eingebracht, und die in der Kammer 3 befindlichen gefrorenen Lösungen werden in dem Behälter 1 gefriergetrocknet. Hierbei muss der Lösungsmitteldampf der -vom Betrachter von Figur 1 aus gesehenunteren Komponenten durch die -vom Betrachter von Figur 1 aus gesehen- oberen Komponenten hindurchsublimieren.

Nach Beendigung des Gefriertrockenprozesses liegen in der Kammer 3 des Behälters 1 die gefriergetrockneten aktiven Komponenten W1, W3, W5 getrennt voneinander und übereinander geschichtet vor. Handelt es sich bei dem Behälter 1 wie in Figur 1 dargestellt um eine Phiole, so kann ein Lösungsmittel 17 von außen beispielsweise mittels einer Spritze zugegeben werden. Nach Auflösung der aktiven Komponenten W1, W3, W5 kann die Lösung dann beispielsweise ebenfalls mittels einer Spritze aus der Phiole entnommen und einem Patienten verabreicht werden. Auch in diesem Fall kann die Lösung selbstverständlich auch auf andere in der Medizin übliche Arten dem Patienten verabreicht werden.

Um die Haltbarkeit und Frische der aktiven Komponenten W1, W3, W5 überprüfen zu können, können in den einzelnen gefriergetrockneten Schichten Komponenten integriert sein, die bei Auflösung und Vermischung durch ein Lösungsmittel 17 miteinander reagieren und so eine Chemi- oder Biolumineszenz-Erscheinung hervorrufen. In diesem Fall kann der Benutzer eine Leuchterscheinung beobachten, wenn die aktiven Komponenten W1, W3 und W5 während der Lagerung keine Möglichkeit hatten miteinander zu reagieren. Hat dagegen eine Reaktion stattgefunden, so haben auch die vorzugsweise schneller reagierenden reaktiven Leuchtkomponenten bereits miteinander reagiert, sodass im Fall der Benutzung keine Leuchterscheinung mehr beobachtet werden kann.

Besonders wenn es sich bei den aktiven Komponenten W1, W3, W5 um sehr aktive Wirk- und/oder Hilfsstoffe, die insbesondere bei Reaktionen miteinander eine hohe Reaktionsgeschwindigkeit aufweisen handelt, ist es angezeigt, Schichten neutraler Komponenten N1, N3, N5 zwischen den aktiven Komponenten W1, W3, W5, W7 anzuordnen, wie es in Figur 2 dargestellt ist. Dies kann dadurch geschehen, dass nach dem Schockgefrieren einer Lösung eines Wirk- und/oder Hilfsstoffes W1, W3, W5, W7 zunächst ein Neutralstoff N1, N3, N5 oder eine Lösung eines Neutralstoffs in die erste Kammer 3 des Behälters 1 eingefüllt wird. Sodann wird der Neutralstoff N1, N3, N5 oder die Lösung des Neutralstoffs schockgefroren, und erst dann wird die nächste aktive Komponente W1, W3, W5, W7 eingefüllt. Es ist selbstverständlich, dass nicht notwendigerweise alle aktiven Komponenten W1, W3, W5, W7 durch Schichten von Neutralkomponenten N1, N3, N5 voneinander separiert werden müssen. Es wird im Allgemeinen genügen, nur diejenigen aktiven Komponenten voneinander zu separieren, die mit auf der Zeitskala der Lagerung des Behälters 1 nennenswerter Reaktionsgeschwindigkeit miteinander reagieren. So ist es möglich, beispielsweise die Komponenten W1 und W3 durch eine neutrale Komponente N1 voneinander zu separieren, während beispielsweise die Komponenten W3 und W5 direkt übereinander anordenbar sind, wenn sie keine nennenswerte Reaktionsgeschwindigkeit miteinander aufweisen. Insofern sind hier beliebige Variationen der Reihenfolge von aktiven Komponenten W1, W3, W5, W7 und neutralen Komponenten N1, N3, N5 denkbar. Werden schockgefrorene Neutralkomponente zwischen aktiven Komponenten angeordnet, ist auch hier vorgesehen, dass abschlie-βend nach dem vollständigen Befüllen der Kammer 3 des Behälters 1 alle Komponenten gemeinsam gefriergetrocknet werden.

Die einzelnen Schritte des dargestellten Verfahrens können auch in anderer als der hier geschilderten Reihenfolge ausgeführt werden, wichtig ist nur, dass als Produkt des Verfahrens ein Behälter resultiert, bei dem mindestens zwei gefriergetrocknete Wirk- und/oder Hilfsstoffe W1, W3, W5, W7 gemeinsam in mindestens einer Kammer 3 vorliegen.

Nach allen zeigt sich, dass die hier vorliegende Erfindung ein Verfahren sowie eine Vorrichtung bereitstellt, die es ermöglicht, verschiedene, möglicherweise reaktive, Wirk- und/oder Hilfsstoffe W1, W3, W5, W7 in einer einzelnen Kammer 3 gemeinsam anzuordnen, ohne dass diese miteinander reagieren. Auf diese Weise können komplexe Mehrkammersysteme, bei denen für jede aktive Komponente eine einzelne Kammer vorgesehen ist, vermieden werden. Alternativ müssen nicht mehrere einzelne Phiolen mit den einzelnen Komponenten bereitgestellt werden, was den Mischungsprozess vor dem Verabreichen fehleranfällig und kompliziert macht. Gleichzeitig wird eine sehr gute Haltbarkeit der Wirkkomponenten erreicht. Das Konzept ist dabei äußerst einfach und auf jede handelsübliche Abfüll- und Gefriertrocknungslinie übertragbar.

## Patentansprüche

1. Behälter für medizinische Produkte mit mindestens einer Kammer (3), wobei mindestens zwei Schichten gefriergetrockneter Wirk- und/oder Hilfsstoffe (W1, W3, W5, W7) gemeinsam in der mindestens einen Kammer (3) vorliegen, **dadurch gekennzeichnet, dass** in die mindestens zwei Schichten Komponenten integriert sind, die bei Auflösung und Vermischung durch ein Lösungsmittel (17) miteinander reagieren können und so eine Chemi- oder Biolumiszenz-Erscheinung hervorrufen können.

2. Behälter nach der Anspruch 1, **dadurch gekennzeichnet, dass** die mindestens zwei Wirk- und/oder Hilfsstoffe (W1, W3, W5, W7) durch mindestens einen gefriergetrockneten Neutralstoff(N1, N3, N5) voneinander getrennt vorliegen.

3. Behälter nach Anspruch 1 oder 2 **dadurch gekennzeichnet, dass** die mindestens zwei Wirk- und/oder Hilfsstoffe (W1, W3, W5, W7) durch mindestens eine Schicht eines gefriergetrockneten Neutralstoffs (N1, N3, N5) voneinander getrennt vorliegen.

4. Behälter nach einem der vorhergehenden Ansprüche, **dadurch gekennzeichnet, dass** der Behälter (1) eine Spritze, Karpule, ein Doppel- oder Mehrkammersystem, eine Phiole, ein Infusionsbeutel oder eine Infusionsflasche ist.

5. Verfahren zur Herstellung eines Behälters (1) nach einem der Ansprüche 1 bis 4, **gekennzeichnet durch** die folgenden Schritte, deren Reihenfolge auch variiert werden kann:
- Bereitstellen eines Behälters (1) für medizinische Produkte mit mindestens einer Kammer (3);
- Einfütten einer Lösung eines ersten Wirk- und/oder Hilfsstoffs in die mindestens eine Kammer (3);
- Schockgefrieren der ersten Lösung;
- Einfüllen einer weiteren Lösung eines Wirk- und/oder Hilfsstoffs In die mindestens eine Kammer (3);
- Schockgefrieren der weiteren Lösung;
- gegebenenfalls Wiederholung der letzten beiden Schritte, bis eine gewünschte Anzahl Wirk- und/oder Hilfsstoffe (W1, W3, W5, W7)in der mindestens einen Kammer (3) vorliegt;
- Gefriertrocknen der gefrorenen Lösungen in dem Behälter (1),
- wobei die gefrorenen Lösungen in dem Behälter Schichten bilden und
- wobei in den einzelnen gefriergetrockneten Schichten Komponenten integriert sind,
- die bei Auflösung und Vermischung **durch** ein Lösungsmittel miteinander reagieren und so eine Chemie- oder Biolumineszenz-Erscheinung hervorrufen.

6. Verfahren nach Anspruch 5 **dadurch gekennzeichnet, dass** nach dem Schockgefrieren einer Lösung eines Wirk- und/oder hilfsstoffes (W1, W3, W5, W7) und vor dem Einfüllen einer weiteren Lösung eines Wirk- und/oder Hilfsstoffes (W1, W3, W5, W7) ein Neutralstoff (N1, N3, N5), vorzugsweise eine Lösung eines Neutralstoffs (N1, N3, N5), in die mindestens eine Kammer (3) eingefüllt wird, wobei der Neutralstoff (N1, N3, N5) oder dessen Lösung nach dem Einfüllen schockgefroren wird.

## Claims

1. Container for medical products which has at least one chamber (3), wherein at least two layers of lyophilized active substances and/or auxiliary substances (W1, W3, W5, W7) are jointly present in the at least one chamber (3), **characterized in that** components are integrated in the at least two layers, which components, when dissolved and mixed by a solvent (17), can react with each other and thus can generate a chemiluminescence or bioluminescence phenomenon.

2. The container according to claim 1, **characterized in that** the at least two active substances and/or auxiliary substances (W1, W3, W5, W7) are present separated from each other by at least one lyophilized neutral substance (N1, N3, N5).

3. The container according to claim 1 or claim 2, **characterized in that** the at least two active substances and or auxiliary substances (W1, W3, W5, W7) are present separated from each other by at least one layer of a lyophilized neutral substance (N1, N3, N5).

4. The container according to any one of the preceding claims, **characterized in that** the container (1) is a syringe, carpule, a dual- or multi-chamber system, a vial, an infusion bag or an infusion bottle.

5. Method for producing a container (1) according to any one of the claims 1 to 4, **characterized by** the following steps, the sequence of which can also be varied:
- providing a container (1) for medical products which has at least one chamber (3);
- filling a solution of a first active substance and/or auxiliary substance into the at least one chamber (3);
- quick-freezing the first solution;
- filling a further solution of an active substance and/or auxiliary substance into the at least one chamber (3);
- quick-freezing the further solution;
- if necessary, repeating the last two steps until a desired number of active substances and/or auxiliary substances (W1, W3, W5, W7) is present in the at least one chamber (3);
- lyophilizing the frozen solutions in the container (1);
- wherein the frozen solutions form layers in the container and
- wherein components are integrated in the discrete lyophilized layers, which components, when dissolved and mixed by a solvent, react with each other and, thus, generate a chemiluminescence or bioluminescence phenomenon.

6. The method according to claim 5, **characterized in that** after quick-freezing a solution of an active substance and/or auxiliary substance (W1, W3, W5, W7) and prior to filling in a further solution of an active substance and/or auxiliary substance (W1, W3, W5, W7), a neutral substance (N1, N3, N5), preferably a solution of a neutral substance (N1, N3, N5), is filled into the at least one chamber (3), wherein the neutral substance (N1, N3, N5) or its solution is quick-frozen after the filling.

## Revendications

1. Contenant pour produits médicaux ayant au moins un compartiment (3), au moins deux couches d'agents et/ou excipients lyophilisés (W1, W3, W5, W7) se trouvant ensemble dans le au moins un compartiment (3), **caractérisé en ce que** des composants sont intégrés dans les au moins deux couches, lesquels peuvent réagir les uns avec les autres lors de la dilution et du mélange par un solvant (17) et peuvent ainsi déclencher un phénomène de chimiluminescence ou de bioluminescence.

2. Contenant selon la revendication 1, **caractérisé en ce que** les au moins deux agents et/ou excipients (W1, W3, W5, W7) se trouvent séparés l'un de l'autre par au moins une matière neutre lyophilisée (N 1, N3, N5).

3. Contenant selon la revendication 1 ou 2, **caractérisé en ce que** les au moins deux agents et/ou excipients (W1, W3, W5, W7) se trouvent séparés l'un de l'autre par au moins une couche d'une matière neutre lyophilisée (N1, N3, N5).

4. Contenant selon l'une des revendications précédentes, **caractérisé en ce que** le contenant (1) est une seringue, une carpule, un système à compartiment double ou à compartiments multiples, une fiole, un sac de perfusion ou une bouteille de perfusion.

5. Procédé de fabrication d'un contenant (1) selon l'une des revendications 1 à 4, **caractérisé par** les étapes suivantes dont on peut également faire varier la séquence :
- préparation d'un contenant (1) pour produits médicaux présentant au moins un compartiment (3) ;
- ajout d'une solution d'un premier agent et/ou excipient dans le au moins un compartiment (3) ;
- congélation rapide de la première solution ;
- ajout d'une seconde solution d'un agent et/ou excipient dans le au moins un compartiment (3) ;
- congélation rapide de la seconde solution ;
- le cas échéant, réitération des deux dernières étapes jusqu'à ce qu'un nombre souhaité d'agents et/ou d'excipients (W1, W3, W5, W7) soient présents dans le au moins un compartiment (3) ;
- lyophilisation des solutions congelées dans le contenant (1),
- les solutions congelées formant des couches dans le contenant et
- des composants étant intégrés dans les différentes couches lyophilisées,
- lesquels réagissent les uns avec les autres lors de la dilution et du mélange par un solvant et déclenchent ainsi un phénomène de chimiluminescence ou de bioluminescence.

6. Procédé selon la revendication 5, **caractérisé en ce que**, après la congélation rapide d'une solution d'un agent et/ou excipient (W1, W3, W5, W7) et avant l'ajout d'une seconde solution d'un agent et/ou excipient (W1, W3, W5, W7), une matière neutre (N1, N3, N5), de préférence une solution d'une matière neutre (N1, N3, N5), est ajoutée dans le au moins un compartiment (3), la matière neutre (N 1, N3, N5) ou sa solution étant congelée rapidement après l'ajout.
